# EUROPÄISCHE PATENTSCHRIFT

(11) **EP 2 510 967 B1**
(45) Veröffentlichungstag und Bekanntmachung des Hinweises auf die Patenterteilung: **17.12.2014**
(21) Anmeldenummer: 12164052.8
(22) Anmeldetag: 13.04.2012
(51) Int. Cl.: A61M 16/00

(54) **HF-Modul für ein Beatmungsgerät zur Hochfrequenzbeatmung insbesondere von neonatalen und pädiatrischen Patienten, Beatmungsgerät**
HF module for a ventilator device for high-frequency ventilation, in particular of neonatal and paediatric patients, ventilation device
Module HF pour un appareil respiratoire destiné à la respiration à haute fréquence, en particulier chez les patients nouveau-nés et pédiatriques, ainsi que l'appareil respiratoire

(30) Priorität: 15.04.2011 DE 202011000890 U
(43) Veröffentlichungstag der Anmeldung: 17.10.2012
(73) Patentinhaber: Heinen & Löwenstein GmbH & Co. KG, 56130 Bad Ems (DE)
(72) Erfinder: Susdorf, Heribert, 56566 Neuwied (DE)
(74) Vertreter: Patentanwälte Bauer Vorberg Kayser

(56) Entgegenhaltungen:
- DE-T2- 69 318 337
- US-A1- 2007 101 999

## Beschreibung

Die Erfindung betrifft ein HF-Modul zur Hochfrequenzbeatmung insbesondere von neonatalen und pädiatrischen Patienten und ein Beatmungsgerät.

Aus dem Stand der Technik sind Hochfrequenzmodule, sogenannte HF-Module zur Hochfrequenzbeatmung beziehungsweise Hochfrequenzoszillation ("HFO") hinlänglich bekannt. Bei dieser Beatmungstechnik wird ein hoher kontinuierlicher alveolärer Distentionsdruck mit Hilfe eines hohen Gasflusses im Beatmungssystem aufgebaut. Hierbei versetzt ein Oszillator, der als Kolben oder Membranantrieb ausgebildet sein kann, das Beatmungsgas in oszillierende Schwingungen. Typisch werden Frequenzen von 5 bis 20 Herz aufgeprägt.

In der Regel wird dieses Verfahren bei Kindern und Neugeborenen angewendet, die am Atemnotsyndrom des Neugeborenen leiden. Jedoch auch bei akutem Lungenversagen bei Erwachsenen wird HFO angewandt.

Aus der DE 101 00 838 A1 geht eine Einrichtung zur künstlichen Beatmung mittels Hochfrequenzoszillation hervor, die einen Betriebssteuerblock aufweist, der eine Beatmungsmengenaufrechterhaltungsfunktion zum Steuern einer durch ein Gebläse festgelegten Amplitude umfasst, so dass die Beatmungsmenge zu einem Zeitpunkt für den Patienten auf einem konstanten Wert gehalten wird, wenn die Oszillationsfrequenz geändert wird.

Weiterhin geht aus der DE 20 2007 010 896 U1 ein HFO-Modul hervor, welches HFO-Modul als Ergänzung zu einem Beatmungsgerät zur hochfrequenten Beatmung insbesondere von Neu- oder Frühgeborenen mit einem veränderlichen Gasvolumen. Das HFO-Modul weist einen Oszillator mit mindestens zwei, zueinander gegenläufig oszillierend ausgebildeten Baugruppen zur Veränderung des Gasvolumens aufweist, wodurch es geringe Geräuschemissionen im Betrieb verursacht.

Ein HF-Modul gemäß dem Obergriff des Anspruchs 1 ist aus DE 693 18 337 T2 bekannt.

Die aktuelle internationale Norm für Beatmungsgeräte IEC 60601-2-12 verlangt, dass alle luftführende Teile von Medizinprodukten im Bereich der Patientenbeatmung steril produziert und auch im Betrieb sterilisierbar oder austauschbar sein müssen. Dies ist bei den bisher bekannten Beatmungsgeräten mit HF-Modul kaum oder nur mit hohen Aufwand möglich, da der Aufbau der HF-Module in den Beatmungsgeräten sehr komplex gestaltet ist.

Aufgabe der vorliegenden Erfindung ist es somit, ein verbessertes HF-Modul beziehungsweise ein verbessertes Beatmungsgerät zur Verfügung zu stellen. Insbesondere ist ein HF-Modul gesucht, dass die Anforderungen an eine ausreichende Reinigbarkeit beziehungsweise Sterilität von luftführenden Teilen erfüllt.

Die Aufgabe wird erfindungsgemäß mittels eines HF-Moduls nach Anspruch 1 und einem Beatmungsgerät nach Anspruch 16 gelöst. Weitere vorteilhafte Ausgestaltungen sind aus der nachfolgenden Beschreibung sowie den Unteransprüchen zu entnehmen. Die einzelnen Merkmale der beschriebenen Ausgestaltungen sind jedoch nicht auf diese beschränkt, sondern können untereinander und mit anderen Merkmalen zu weiteren Ausgestaltungen verknüpft werden.

Es wird ein HF-Modul zur Hochfrequenzbeatmung insbesondere von neonatalen und pädiatrischen Patienten vorgeschlagen, wobei das HF-Modul in der Regel als Ergänzungsmodul zu einem bestehenden Beatmungsgerät ausgebildet ist. Das HF-Modul umfasst zumindest eine Antriebseinheit zur Erzeugung hochfrequenter Schwingungen und zumindest ein HF-Gehäuse, wobei das HF-Gehäuse eine Zugangsöffnung, die insbesondere als Schlauchanschluss ausgebildet sein kann, und eine Membran umfasst. Das HF-Gehäuse ist an die Antriebseinheit koppelbar, dergestalt dass die Membran mittels der Antriebseinheit auslenkbar ist. Weiterhin weist das HF-Gehäuse ein Koppelmittel auf, mit dem das HF-Gehäuse lösbar an das HF-Modul angebunden werden kann. Insbesondere kann das Koppelmittel dazu ausgebildet sein, das HF-Gehäuse lösbar an die Antriebseinheit anzubinden.

Vorteil des vorgeschlagenen HF-Moduls ist, dass das luftführende HF-Gehäuse von dem HF-Modul vollständig getrennt werden kann. Dies ermöglicht in einer Ausgestaltung eine separate Reinigung und/oder Sterilisation des HF-Gehäuses. In einer weiteren Ausgestaltung ist vorgesehen, dass das HF-Gehäuse nach einer maximalen Nutzungsdauer oder bei einem Patientenwechsel entfernt und entsorgt beziehungsweise recycelt wird. Das gebrauchte HF-Gehäuse kann dann gegen ein neues und/oder ein sterilisiertes HF-Gehäuse ausgetauscht werden.

In einer besonders vorteilhaften ersten Ausgestaltung ist das HF-Gehäuse werkzeugfrei an das HF-Modul anbindbar, das heißt, dass die Kopplung und insbesondere auch die Entkopplung des HF-Gehäuses an die Antriebseinheit ohne Werkzeuge erfolgen kann. In einer weiteren Ausgestaltung ist vorgesehen, dass ein Werkzeug oder ein Schlüssel für die Anbindung und/oder das Lösen des HF-Gehäuses notwendig ist. Dies ist insbesondere dann sinnvoll, wenn ein unbefugtes oder versehentliches Lösen des HF-Gehäuses vermieden werden soll. Zur Kopplung ist ein Koppelmittel am erfindungsgemäßen HF-Modul vorgesehen, wobei in einer bevorzugten Ausgestaltung vorgesehen ist, dass das Koppelmittel eine Bajonettkupplung, eine Spannkupplung, eine Schraubkupplung, eine Rastkupplung oder eine Steckkupplung aufweist. Mittels des Koppelmittels, deren Gegenstück von dem HF-Modul selbst, insbesondere der Antriebseinheit, oder an diesem ausgebildet wird, kann das HF-Gehäuse mit der Antriebseinheit gekoppelt werden. Bevorzugt ist das Koppelmittel ohne Verwendung von Werkzeug von einem Benutzer des Beatmungsgeräts, mit dem das erfindungsgemäße HF-Modul verwendet wird, lösbar. Alternativ kann das Koppelmittel nur unter Verwendung von Spezialwerkzeug wie einem codierten Schlüssel zu öffnen sein. Dies kann z.B. über eine integriert oder separat ausgebildete Verriegelung realisiert werden. Auch bei einem ohne Verwendung von Werkzeug lösbaren Koppelmittel kann eine solche Verriegelung realisiert werden, beispielsweise in Form einer mechanischen Abdeckung, die das Koppelmittel durch zumindest teilweise mechanische Überdeckung dem Zugriff eines Benutzers entzieht. Eine solche Abdeckung kann auch mit einem Koppelmittel, welches nur unter Verwendung von Spezialwerkzeug wie einem codierten Schlüssel geöffnet werden kann, zusätzlich vorgesehen sein.

In des erfindungsgemäßen Ausgestaltung ist vorgesehen, dass das HF-Modul zumindest zwei Antriebseinheiten aufweist, die jeweils eine Membran entgegengesetzt oder gleichgerichtet zueinander auslenken. Vorzugsweise findet die Auslenkung der Membrane synchron oder entgegengesetzt synchron statt. Besonders vorteilhaft sind die Membranen derart angeordnet und angesteuert, dass diese jeweils einen gemeinsamen vorzugsweise kumulierbaren Schalldruckimpuls auslösen. Vorteilhaft sind mehrere HF-Gehäuse kommunizierend miteinander verbunden. In einer Ausgestaltung ist des Weiteren vorgesehen, dass Ventile, insbesondere Rückschlagventile, zwischen den miteinander verbundenen HF-Gehäusen vorgesehen sind.

In einer weiteren Ausgestaltung ist vorgesehen, dass mehr als zwei HF-Module vorgesehen sind. Weiterhin sieht eine Ausgestaltung vor, dass ein HF-Modul zwei oder mehr als zwei Antriebseinheiten umfasst. So kann das HF-Gehäuse eine, zwei oder mehr als zwei Membrane aufweisen, die durch die Antriebseinheiten ausgelenkt werden, um innerhalb des HF-Gehäuses einen Impuls zu erzeugen. Bevorzugt weisen die Antriebseinheiten zumindest paarweise kollineare Betätigungsrichtungen auf. Besonders bevorzugt wird jeweils ein solches Paar von Antriebseinheiten gegenläufig betrieben. In einer besonders vorteilhaften Ausgestaltung ist vorgesehen, dass an jede Antriebseinheit ein HF-Gehäuse anbindbar ist.

Besonders vorteilhaft ist, wenn das HF-Gehäuse derart am HF-Modul angeordnet ist, dass dieses von außen leicht zugänglich ist. Insbesondere ist vorgesehen, dass das HF-Gehäuse bzw. die HF-Gehäuse lateral des HF-Moduls weiterhin bevorzugt lateral des Beatmungsgerätes angeordnet ist/sind.

Die Antriebseinheiten sind räumlich aneinander angrenzend angeordnet, dergestalt dass sich die Antriebseinheiten im Betrieb des HF-Moduls mechanisch aneinander abstützen können. Hierzu sind die Längsachsen der Antriebseinheiten, welche durch deren Betätigungsrichtungen definiert werden, kollinear, bevorzugt entlang einer gemeinsamen Achse ausgerichtet, orientiert.

Die Antriebseinheit weist in einer Ausgestaltung einen Membranantrieb auf. Weiterhin vorteilhaft weist die Antriebseinheit einen vorzugsweise elektrodynamischen Lautsprecher auf. In einer weiteren Ausgestaltung umfasst die Antriebseinheit ein Kolben-Zylinder-System.

In einer weiteren Variante ist vorgesehen, dass die Antriebseinheit jeweils einen Stator und einen Aktor aufweist, wobei sich in einer Ausgestaltung die Statoren zumindest jeweils zweier Antriebseinheiten gegenseitig abstützen. Dies hat bei synchroner entgegengesetzter Auslenkung der Aktoren den Vorteil, dass die Gegenkräfte die bei Auslenkung des Aktors auf den Stator wirken von dem entgegengesetzt angeordneten Stator erwidert werden. Das bedeutet, dass die Reaktionskraft durch den entgegengesetzten Stator aufgebracht wird und nicht etwa durch das Gehäuse des Beatmungsgerätes oder des HF-Modules. Die Statoren geraten dadurch nicht oder nur geringfügig in Bewegung, was zu einer Minimierung der Geräuschentwicklung bei einem Betrieb des HF-Moduls führt. Auch wird die Schwingungsübertragung auf ein Gehäuse des Beatmungsgerätes beziehungsweise des HF-Modules vermieden. In einer Ausgestaltung ist zumindest eine Dämpfung, beispielsweise eine Dämpfungsmatte zwischen den Statoren angeordnet. Vorzugsweise ist die Vorrichtung, welche die Antriebseinheit hält, über eine Dämpfung mit einem Gehäuse und/oder weiteren Teilen des HF-Moduls und/oder des Beatmungsgerätes verbunden.

Unter einem Aktor wird der bewegliche Teil der Antriebseinheit verstanden, beispielsweise umfasst der Aktor bei einem elektrodynamischen Lautsprecher die Schwingspule insbesondere in Wirkverbindung mit der Lautsprechermembran. Unter einem Stator wird der unbewegliche Teil der Antriebseinheit verstanden, das heißt der Teil oder die Teile, der die Gegenkräfte bei Auslenkung des Aktors aufnimmt. Bei einem elektrodynamischen Lautsprecher zum Beispiel umfasst der Stator den Permanentmagneten. Der Stator kann auch die Antriebseinheit haltende Vorrichtungen aufweisen. In einer bevorzugten Ausgestaltung ist eine Antriebseinheit-haltende Vorrichtung vorgesehen, die zumindest zwei Antriebseinheiten hält. Vorzugsweise ist die Antriebseinheit-haltende Vorrichtung einteilig oder einstückig ausgestaltet.

In einer besonders vorteilhaften Ausgestaltung ist vorgesehen, dass ein Innenraum des HF-Gehäuses von der Antriebseinheit hermetisch getrennt ist. Die Membran des HF-Gehäuses wird vorzugsweise durch den Aktor der Antriebseinheit ausgelenkt. Dies hat den Vorteil, dass die Antriebseinheit nicht sterilisiert werden braucht, da sie nicht in Kontakt mit Gasen kommt, die einem Patienten zugeführt werden.

In einer Ausgestaltung, bei der die Antriebseinheit einen elektrodynamischen Lautsprecher umfasst, ist vorgesehen, dass durch die Auslenkung der Lautsprechermembran ein zwischen der Membran des HF-Gehäuses und der Lautsprechermembran angeordnetes Luftvolumen in Schwingung versetzt wird und die Membran des HF-Gehäuses bewegt. Weiterhin sieht eine Ausgestaltung vor, dass die Lautsprechermembran zumindest teilweise an der Membran des HF-Gehäuses anliegt.

Eine weitere Variante sieht vor, dass die Antriebseinheit unmittelbar oder über einen mechanischen Koppelmechanismus mit der Membran des HF-Gehäuses in mechanischen Kontakt gebracht wird, um diese auszulenken. Die Antriebseinheit kann beispielsweise als elektromagnetischer, piezoelektrischer, pneumatischer oder hydraulischer Aktuator ausgebildet sein. Der Koppelmechanismus kann ein Hebelmechanismus sein, der ggf. zusätzlich eine mechanische Über- oder Untersetzung der von der Antriebseinheit erzeugten Stellbewegung bewirkt.

In einer Ausführungsform ist vorgesehen, dass das HF-Gehäuse ein Material umfasst, das ausgewählt ist aus einer Gruppe zumindest umfassend Polysulfon, Polyimide, Polyetherimide, Polyethylene, Polypropylen, Polyurethane und/oder Polyehylenterphtalat. Besonders bevorzugt sind Materialen, die autoklavierbar sind. Weiterhin bevorzugt sind Materialien, die temperaturbeständig bis zumindest etwa 121°C vorzugsweise zumindest etwa 134 °C, sind. Unter temperaturbeständig wird verstanden, dass das Material bei einer definierten Temperaturbelastung über eine gewisse Zeitdauer, vorzugsweise zumindest etwa 20 Minuten, weiterhin bevorzugt zumindest etwa 60 Minuten keine nicht reversible chemische oder geometrische Materialveränderung erfährt. Temperaturbedingte Ausdehnungen sind tolerierbar. Beispielsweise ist ein Schmelzen, oder ein Erweichen des Materials beispielsweise bei einem Autoklaviervorgang, das beispielsweise zum Beulen oder Knicken des Materials aufgrund des Eigengewichts führt, ein Zeichen dafür, dass das Material nicht temperaturbeständig ist.

Soweit in der vorliegenden Erfindung der Begriff "etwa" verwendet wird, gibt dieser einen Toleranzbereich an, den der auf dem vorliegenden Gebiet tätige Fachmann für üblich betrachtet. Insbesondere ist unter dem Begriff "etwa" ein Toleranzbereich von bis +/-20%, bevorzugt bis +/-10% zu verstehen.

In einer weiteren Ausgestaltung ist vorgesehen, dass die Membran des HF-Gehäuses ein Material umfasst, ausgewählt aus einer Gruppe zumindest umfassend Polyester, Polytetrafluoräthylen, Silikon und/oder Polyvinylchlorid. Vorzugsweise ist die Membran autoklavierbar, weiterhin bevorzugt temperaturbeständig zumindest bis vorzugsweise etwa 121 °C, weiter bevorzugt zumindest bis etwa 134 °C.

In einer weiteren Ausführungsform ist vorgesehen, dass das HF-Gehäuse ein Spritzgussteil aufweist. Vorzugsweise ist die Membran angespritzt. In einer weiteren Ausgestaltung ist die Membran angeklebt, angeschweißt oder durch einen Klemmring oder auf sonstige geeignete Weise lösbar mit dem Gehäuse verbunden, so dass die Membran als Einweglösung austauschbar ist. Eine Autoklavierbarkeit der Einwegmembran ist dann nicht erforderlich. Weiterhin sieht eine vorteilhafte Variante vor, dass die Membran mit dem Gehäuse einstückig verbunden ist.

Weiterhin sieht eine vorteilhafte Variante vor, dass das HF-Gehäuse - abgesehen von der Zugangsöffnung - vollständig geschlossen ist. Vorzugsweise ist die Zugangsöffnung bzw. der Schlauchanschluss die einzige Öffnung des HF-Gehäuses. In einer bevorzugten Ausgestaltung bildet das HF-Gehäuse im betätigten Zustand des Koppelmittels - abgesehen von der Zugangsöffnung - einen keimdicht abgedichteten Innenraum aus. Besonders bevorzugt ist das HF-Gehäuse so ausgebildet, dass es einen keimdicht abgedichteten Innenraum ausbildet, der auch im gelösten Zustand des Koppelmittels - abgesehen von der Zugangsöffnung - ausschließlich über die Zugangsöffnung zugänglich ist.

In einer weiteren Ausgestaltung ist vorgesehen, dass die Zugangsöffnung bzw. der Schlauchanschluss ein Ventil aufweist, das insbesondere erst bei bzw. durch Anschließen eines Schlauches geöffnet wird.

Eine weitere Ausführungsform sieht vor, dass die Zugangsöffnung des HF-Gehäuses als verschließbare, vorzugsweise luftdicht verschließbare Öffnung ausgebildet ist. Insbesondere ist ein Deckel vorgesehen, mit dem die Zugangsöffnung luft- und keimdicht verschließbar ist. Dies hat den Vorteil, dass für einen Sterilisierungsvorgang die Zugangsöffnung geöffnet wird und das sterilisierende Medium leicht in den Innenraum des HF-Gehäuses dringen kann. Nach dem Sterilisieren wird die Zugangsöffnung des HF-Moduls verschlossen, wodurch das HF-Modul steril lagerfähig ist. Besondere Vorteile ergeben sich weiterhin, wenn die Membran des HF-Gehäuses in den Deckel der Zugangsöffnung integriert ist. Auch hier kann die Membran einstückig mit dem Deckel ausgebildet oder als separates Teil ausgebildet sein. Insbesondere kann im Deckel ein Klemmring vorgesehen sein, mit dem eine austauschbare Einweg-Membran, die aus einem nichtautoklavierbaren Kunststoff bestehen kann, am Deckel festgelegt wird.

Ein weiterer Gedanke der Erfindung sieht ein Beatmungsgerät umfassend ein oben beschriebenes HF-Modul vor.

Weiterhin wird ein Verfahren zur Reinigung luftführender Teile eines HF-Moduls beschrieben, wobei das HF-Modul zumindest eine Antriebseinheit und zumindest ein luftführendes HF-Gehäuse umfasst, wobei das HF-Gehäuse eine beispielsweise als Schlauchanschluss ausgebildete Zugangsöffnung und eine Membran umfasst, wobei das lösbar an die Antriebseinheit angebundene HF-Gehäuse vom HF-Modul getrennt und in einem weiteren Schritt sterilisiert wird.

Vorteil dieses Verfahrens ist, dass die Reinigung und Sterilisation nicht am Gerät selbst erfolgen muss. Vielmehr ist es möglich die luftführenden Teile des HF-Modules, das heißt das HF-Gehäuse und gegebenenfalls die daran angeschlossenen Schläuche vom Gerät zu trennen und dann beispielsweise mittels eines Antiseptikums oder in einem Autoklaven zu sterilisieren. Die Sterilisation des HF-Gehäuses kann beispielsweise mittels Erhitzen (insbesondere im feuchten Zustand), durch Zuführen von trockenen oder flüssigen Antiseptika wie beispielsweise Wasserstoffperoxid, Ozon, Formaldehyd oder Peressigsäure, und/oder durch Plasma- oder Strahlenexposition erfolgen.

In einer vorteilhaften Variante ist vorgesehen, dass das HF-Gehäuse durch ein neues und/oder bereits sterilisiertes HF-Gehäuse ausgetauscht wird. Insbesondere kann das HF-Gehäuse als Einmal-Produkt ausgestaltet sein, das nach der Verwendung entsorgt beziehungsweise recycelt wird.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
- Fig. 1: ein HF-Modul;
- Fig. 2: ein HF-Gehäuse;
- Fig. 3: eine schematische Ansicht eines Beatmungsgerätes;
- Fig. 4: eine schematische Schnittansicht eines Teiles eines HF-Moduls;
- Fig. 5: eine weitere schematische Schnittansicht eines HF-Moduls; und
- Fig. 6:: eine schematische Teilschnittansicht eines weiteren HF-Moduls.

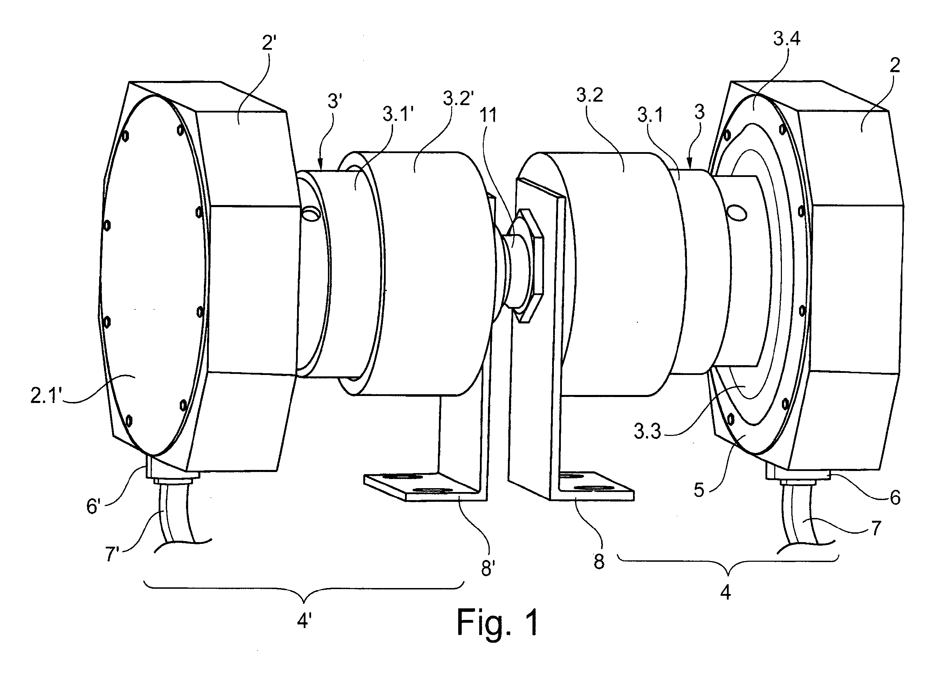

Figur 1 zeigt ein HF-Modul 1, welches zwei HF-Gehäuse 2, 2' sowie zwei Antriebseinheiten 3, 3' aufweist. Jeweils ein HF-Gehäuse 2, 2' und eine Antriebseinheit 3, 3' bilden einen Modulteil 4, 4'. Die Antriebseinheiten 3, 3' sind jeweils als elektrodynamische Lautsprecher mit Aktor 3.2, 3.2', Stator 3.1, 3.1' und Korb 3.3 ausgebildet. In Figur 1 ist der Korb 3.3 der auf der rechten Seite der Figur 1 abgebildeten Antriebseinheit 3 zu erkennen. Die Antriebseinheiten 3, 3' sind mittels Koppelmitteln 5 mit den HF-Gehäusen 2, 2' verbunden. Die Koppelmittel 5 sind in dieser Ausgestaltung als durch Schraubenlöcher in den Flanschen 3.4 der Körbe 3.3 der Lautsprecher hindurchgreifende Schrauben ausgebildet. Diese Schrauben verbinden den jeweiligen Korb 3.3 lösbar mit dem benachbarten HF-Gehäuse 2, 2'. Zwischen den beiden Aktoren 3.2, 3.2' der entgegengesetzt zueinander angeordneten Modulteile 4, 4' ist eine Brücke 14 angeordnet, über die sich die beiden Aktoren 3.2, 3.2' mechanisch aneinander abstützen.

Weiterhin ist Figur 1 zu entnehmen, dass die HF-Gehäuse 2, 2' Schlauchanschlüsse 6 aufweisen, die (permanente) Zugangsöffnungen zum Innenraum der hohl ausgebildeten HF-Gehäuse 2, 2' bereitstellen. Die Schlauchanschlüsse 6, 6' beider HF-Gehäuse 2, 2' sind jeweils mit einem Schlauch 7, 7' verbunden.

Jedes der beiden gezeigten HF-Gehäuse 2, 2' weist neben der Zugangsöffnung noch einen Deckel 2.1 auf, der geöffnet werden kann oder entnehmbar ausgebildet ist. Durch Öffnen der Deckel 2.1 wird jeweils eine weitere (temporäre) Zugangsöffnung mit großem Querschnitt zum Innenraum der HF-Gehäuse 2, 2' bereitgestellt, so dass der Innenraum einfach für einen Sterilisationsprozess zugänglich ist.

In der gezeigten Ausgestaltung ist an jeder Antriebseinheit 3, 3' eine Antriebseinheit-haltende Vorrichtung 8, 8' in Form eines Metallwinkels angeordnet ist. Diese sind fest mit der jeweiligen zugeordneten Antriebseinheit 3, 3' verbunden. Insbesondere ist vorgesehen, dass die Antriebseinheit-haltenden Vorrichtungen 8, 8' jeweils mit der zugeordneten Antriebseinheit 3, 3' verschraubt, verschweißt oder verspannt sind.

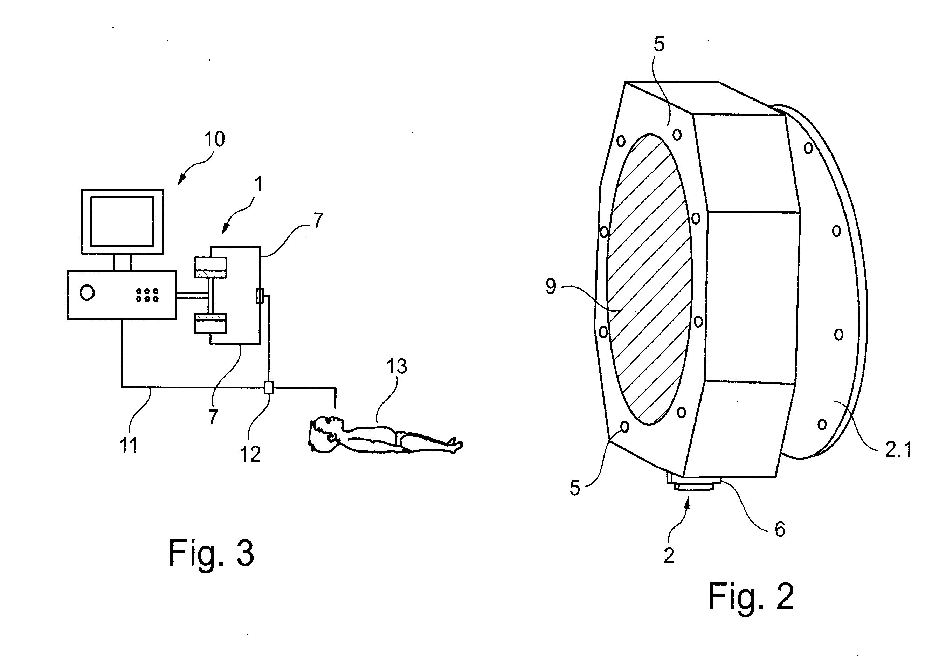

Figur 2 zeigt ein HF-Gehäuse 2, das von dem HF-Modul 1 getrennt ist. Deutlich zu erkennen sind die hier als durch Schraublöcher hindurchgreifende Schrauben ausgestalteten Koppelmittel 5 sowie die Membran 9, die durch die Antriebseinheit 3 auslenkbar ist. Weiterhin ist der Figur 2 zu entnehmen, dass das HF-Gehäuse 2 einen Schlauchanschluss 6 aufweist. Das von dem HF-Modul 1 getrennte HF-Gehäuse 2 kann ohne weiteres gereinigt und / oder vorzugsweise in einem Autoklaven sterilisiert werden. Bevorzugt ist die geometrische Gestaltung des HF-Gehäuses 2 in der Art, dass ein Wasserdampf während der Sterilisation ohne Probleme in den Innenraum des HF-Gehäuses 2 eindringen kann. Weiterhin bevorzugt sind Kanten, die sich im Innenraum befinden derart ausgestaltet, vorzugsweise abgerundet, dass eine Kondensation im Bereich der innenliegenden Ecken vermieden wird oder zumindest Kondensationströpfchen den Sterilisationsvorgang nicht wesentlich behindern.

In der gezeigten Ausgestaltung ist vorgesehen, dass das HF-Gehäuse einen Deckel 2.1 aufweist, der das HF-Gehäuse 2 im Betrieb luftdicht verschließt, jedoch bei einem Autoklaviervorgang geöffnet wird, so dass der Wasserdampf ungehindert in den Innenraum des HF-Gehäuses 2 eindringen kann. Der Deckel 2.1 kann aufgeschraubt, verschraubt, eingerastet oder sonst wie mit dem HF-Gehäuse 2 verbunden sein. Vorzugsweise findet sich zwischen Deckel 2.1 und HF-Gehäuse 2 eine hier nicht gezeigte vorzugsweise sterilisierbare Dichtung. In einer weiteren vorteilhaften Ausgestaltung ist vorgesehen, dass der Deckel mittels eines Filmscharniers an das HF-Gehäuse 2 angelenkt ist.

Figur 3 zeigt eine schematische Ansicht eines Beatmungsgerätes 10 mit einem HF-Modul 1. Das HF-Modul 1 mit zwei Modulteilen 4, 4' ist über zumindest zwei vorzugsweise gleich lange Schläuche 7, 7' mit einem Beatmungsschlauch 11 wirkverbunden. In der hier gezeigten Ausgestaltung wird die Hochfrequenzventilation, die von dem HF-Modul 1 ausgeht, über eine Kupplung 12 in den Beatmungsschlauch 11 eingekoppelt. Der Beatmungsschlauch 11 ist mit dem insbesondere intubierten Patienten 13 verbunden.

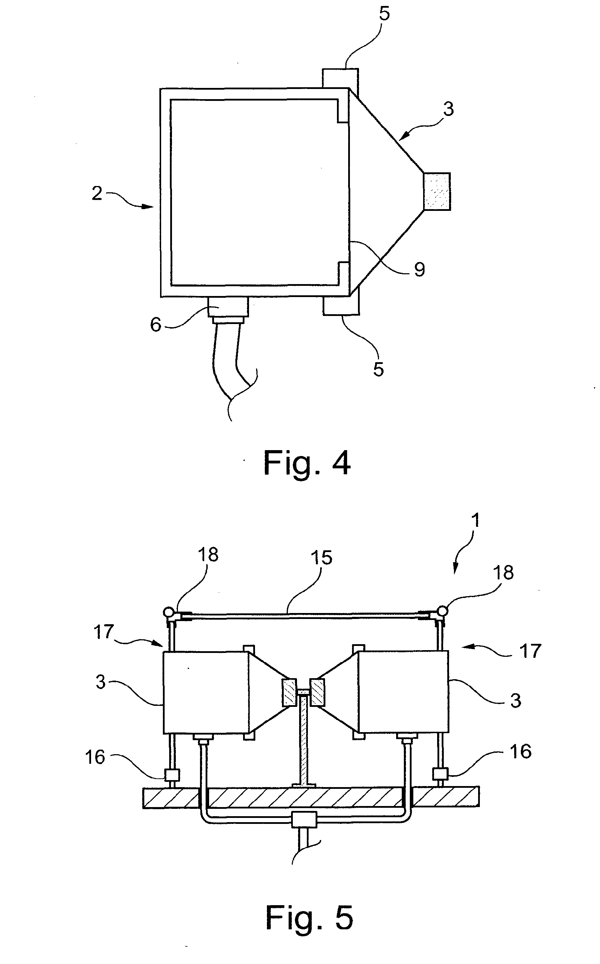

Figur 4 zeigt eine schematische Schnittansicht eines HF-Moduls 1. Zu erkennen ist das HF-Gehäuse 2 mit dem Schlauchanschluss 6 und der Membran 9. Die Membran 9 kann in einer weniger vorteilhaften Ausgestaltung ggf. entfallen. Weiterhin zu erkennen ist die Antriebseinheit 3, die mittels eines Koppelmittels 5, das in der gezeigten Ausführungsform als Bajonettverschluss ausgebildet ist, an die Antriebseinheit 3 gekoppelt ist.

Fig. 5 zeigt eine weitere Variante eines HF-Moduls 1 in einer schematischen Schnittansicht. Das HF-Modul weist ein Gehäuse 15 auf, welches mit einen Verschlussmechanismus 16 ausgestattet ist. Der Verschlussmechanismus dient insbesondere zur Vermeidung eines unbefugten Zugriffes und ist derart ausgestaltet, dass vorzugsweise ein Werkzeug, beispielsweise ein Schlüssel, zur Öffnung verwendet werden muss.

Das Gehäuse 15 weist Aussparungen 17 auf, durch die die HF-Gehäuse 2 sichtbar sind oder das Gehäuse 15 durchdringen. Besonders bevorzugt sind die HF-Gehäuse 2 zumindest teilweise transparent oder transluzent ausgebildet, insbesondere an der von außen sichtbaren Seite. Weiterhin bevorzugt ist vorgesehen, dass die HF-Gehäuse 2 einen Nutzungsindikator aufweisen, der anzeigt, ob die HF-Gehäuse 2 ausgetauscht werden müssen. Beispielsweise reagiert der Nutzungsindikator auf Feuchtigkeit und zeigt diese visuell - beispielsweise durch einen Farbumschlag - an.

Besonders bevorzugt weist das Gehäuse 15 des Beatmungsgeräts 10 Scharniere 18 auf, mittels denen das Gehäuse 15 geöffnet werden kann, wodurch der Zugriff auf die HF-Gehäuse 2 möglich ist. Insbesondere können die Koppelmittel 5 so innerhalb des Gehäuses 15 angeordnet sein, dass sie erst dann betätigbar sind, wenn das Gehäuse 15 geöffnet ist.

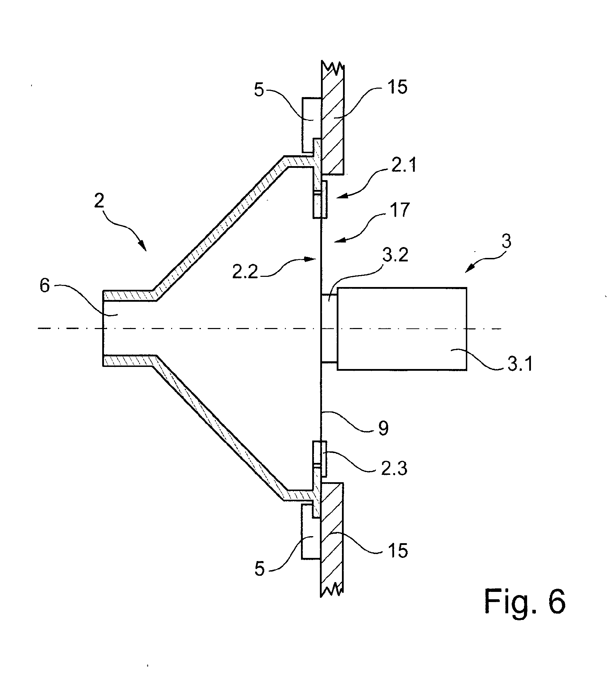

Fig. 6 schließlich zeigt ausschnittsweise ein Ausführungsbeispiel eines Beatmungsgeräts 10. Das Gehäuse 15 des Beatmungsgeräts weist Aussparungen 17 auf, durch die der Stator 3.2 eines elektromagnetischen Linearantriebs, der als Antriebseinheit 3 dient, hindurchgreift. Der Stator 3.2 betätigt die Membran 9 eines HF-Gehäuses 2, welches im Bereich der Aussparung 17 von außen auf das Gehäuse 15 des Beatmungsgeräts 10 aufgesetzt und mechanisch mi diesem verrastet ist. Hierzu ist als Koppelmittel 5 eine mittels Drehung des HF-Gehäuses 2 betätigbare Bajonettkupplung vorgesehen ist. Das HF-Gehäuse 2 ist als autoklavierbares Spritzgussteil ausgebildet, welches einen einstückig angeformten Schlauchanschluss 6 und eine Zugangsöffnung 2.2 mit großem (rundem) Querschnitt für eine einfachen Sterilisierbarkeit aufweist. In die Zugangsöffnung 2.2 eingesetzt ist ein ringförmiger Deckel 2.1, dessen ringförmige Öffnung von einer elastischen Membran 9 überspannt ist. Die Membran 9 ist unverlierbar mit dem ringförmigen Teil des Deckels 2.1. verbunden. Hierzu kann der ringförmige Teil als Spritzgussteil zweiteilig ausgebildet sein, wobei die beiden Teile mit der Membran 9 miteinander verklebt sein können. Der Deckel 2.1. ist bevorzugt abdichtend in die Zugangsöffnung 2.2 einschraubbar.

Soll das Beatmungsgerät sterilisiert werden, so wird ein am Schlauchanschluss 6 befestigter Schlauch 7 gelöst und das HF-Gehäuse 2 durch Betätigung des Bajonettverschlusses vom Gerät 10 getrennt. Danach wird der Deckel 2.1 vom HF-Gehäuse 2 gelöst und entsorgt. Das HF-Gehäuse 2 und alle Atemgas-führenden Schläuche 7, 11 und sonstigen außenliegenden Komponenten werden auf geeignete Weise sterilisiert. Schließlich wird die Zugangsöffnung 2.2 mit einem neuen sterilen Deckel 2.1 mit Membran 9 wieder verschlossen und das HF-Gehäuse 2 wieder am Gehäuse 15 des Beatmungsgeräts 10 angebracht. Ist das HF-Gehäuse 2 aus einem transluzenten Material gefertigt, so kann bei geeigneter Ausbildung der Membran 9 ein eventueller Farbumschlag der Membran 9, der die Notwendigkeit einer Sterilisation des HF-Gehäuses 2 anzeigt, von außen inspiziert werden, wodurch eine besonders einfache Wartung des Geräts 10 ermöglicht wird.

In allen Ausführungsbeispielen, insbesondere bei erfindungsgemäßen Beatmungsgeräten 10 mit zumindest zwei HF-Gehäusen 2, 2', ist es von besonderem Vorteil, wenn Ventile vorgesehen sind, die es erlauben, selektiv einzelne HF-Gehäuse 2 gegenüber den anderen HF-Gehäusen 2' und gegenüber dem Patienten 13 strömungstechnisch abzusperren. Dies ermöglicht ggf. einen Austausch der HF-Gehäuse 2 im laufenden Betrieb des Beatmungsgeräts 10.

### Bezugszeichenliste

- 1: HF-Modul
- 2: HF-Gehäuse
- 2.1: Deckel
- 2.2: Zugangsöffnung
- 2.3: ringförmiges Teil
- 3.: Antriebseinheit
- 3.1: Aktor
- 3.2: Stator
- 3.3: Korb
- 4: Modulteil
- 5: Koppelmittel
- 6: Schlauchanschluss
- 7: Schlauch
- 8: Haltevorrichtung
- 9: Membran
- 10: Beatmungsgerät
- 11: Beatmungsschlauch
- 12: Kupplung
- 13: Patient
- 14: Brücke
- 15: Gehäuse
- 16: Verschlussmechanismus
- 17: Aussparung
- 18: Scharnier

## Patentansprüche

1. HF-Modul (1) für ein Beatmungsgerät zur Hochfrequenzbeatmung insbesondere von neonatalen und pädiatrischen Patienten (13), wobei das HF-Modul (1) zumindest eine Antriebseinheit (3) zur Erzeugung hochfrequenter Schwingungen und zumindest ein HF-Gehäuse (2) umfasst, wobei das HF-Gehäuse (2) eine Zugangsöffnung, die als Schlauchanschluss (6) ausgebildet sein kann, und eine Membran (9) umfasst, wobei das HF-Gehäuse (2) an die Antriebseinheit (3) koppelbar ist, dergestalt dass die Membran (9) mittels der Antriebseinheit (3) auslenkbar ist, wobei das HF-Gehäuse (2) ein Koppelmittel (5) aufweist, mit dem das HF-Gehäuse (2) lösbar an die Antriebseinheit (3) angebunden werden kann, **dadurch gekennzeichnet, dass** das HF-Modul (1) zumindest ein weiteres HF-Gehäuse (2') und zumindest eine weitere Antriebseinheit (3') aufweist, wobei jedem HF-Gehäuse (2, 2') eine Antriebseinheit (3, 3') zugeordnet ist, die auf die Membran (9, 9') des zugeordneten HF-Gehäuses (2, 2') einwirkt, wobei die Betätigungsrichtungen der Antriebseinheiten (3,3') kollinear sind und die Antriebseinheiten (3, 3') gegenläufig betrieben werden, und dass die Antriebseinheiten räumlich aneinander angrenzend angeordnet sind, so dass sich die Antriebseinheiten im Betrieb des HF-Moduls (1) mechanisch aneinander abstützen können.

2. HF-Modul (1) nach Anspruch 1, **dadurch gekennzeichnet, dass** das Koppelmittel (5) dazu ausgebildet ist, das HF-Gehäuse (2) lösbar an die Antriebseinheit (3) anzubinden.

3. HF-Modul (1) nach einem der vorhergehenden Ansprüche, **dadurch gekennzeichnet, dass** das Koppelmittel (5) eine Bajonettkupplung, eine Spannkupplung, eine Schraubkupplung, eine Rastkupplung, einen Flansch oder eine Steckkupplung aufweist.

4. HF-Modul (1) nach Anspruch 3, **dadurch gekennzeichnet, dass** das Koppelmittel (5) von einem Benutzer ohne Werkzeug lösbar ausgebildet ist.

5. HF-Modul (1) nach Anspruch 3 oder 4, **dadurch gekennzeichnet, dass** eine Verriegelung für das Koppelmittel (5) vorgesehen ist, die einen Öffnungs- und einen Verriegelungszustand aufweist, wobei das Koppelmittel (5) ausschließlich im Öffnungszustand der Verriegelung gelöst werden kann.

6. HF-Modul (1) nach Anspruch 5, **dadurch gekennzeichnet, dass** die Verriegelung als Schloss ausgebildet ist.

7. HF-Modul (1) nach einem der Ansprüche 5 und 6, **dadurch gekennzeichnet, dass** die Verriegelung als Abdeckung ausgebildet ist, die das Koppelmittel (5) durch zumindest teilweise Überdeckung dem Zugriff eines Benutzers entzieht.

8. HF-Modul (1) nach einem der vorhergehenden Ansprüche, **dadurch gekennzeichnet, dass** das HF-Gehäuse (2) im betätigten Zustand des Koppelmittels (5) einen keimdicht abgedichteten Innenraum ausbildet.

9. HF-Modul (1) nach Anspruch 1, **dadurch gekennzeichnet, dass** das HF-Gehäuse (2) einen keimdicht abgedichteten Innenraum ausbildet, der auch im gelösten Zustand des Koppelmittels (5) ausschließlich über die Zugangsöffnung zugänglich ist.

10. HF-Modul (1) nach Anspruch 9, **dadurch gekennzeichnet, dass** die Membran (9) einstückig mit dem HF-Gehäuse (2) ausgebildet ist.

11. HF-Modul (1) nach Anspruch 9, **dadurch gekennzeichnet, dass** die Membran (9) unverlierbar mit dem HF-Gehäuse (2) verbunden ist.

12. HF-Modul (1) nach einem der vorhergehenden Ansprüche, **dadurch gekennzeichnet, dass** die Antriebseinheiten (3, 3') jeweils einen Stator (3.1, 3.1') und einen Aktor (3.2, 3.2') aufweisen, wobei sich die Statoren (3.1, 3.1') zumindest jeweils zweier Antriebseinheiten (3) mechanisch aneinander abstützen.

13. HF-Modul (1) nach einem der vorhergehenden Ansprüche, **dadurch gekennzeichnet, dass** das HF-Gehäuse (2) ein Material umfasst ausgewählt aus einer Gruppe zumindest umfassend Polysulfon, Polyimide, Polyetherimide, Polyethylene, Polypropylen, Polyurethane und/oder Polyethylenterphtalat.

14. HF-Modul (1) nach einem der vorhergehenden Ansprüche, **dadurch gekennzeichnet, dass** die Membran (9) des HF-Gehäuses (2) ein Material umfasst ausgewählt aus einer Gruppe zumindest umfassend Polyester, Polytetrafluorethylen, Silikon und/oder Polyvinylchlorid.

15. HF-Modul (1) nach einem der vorhergehenden Ansprüche, **dadurch gekennzeichnet, dass** das HF-Gehäuse (2) ein Spritzgussteil aufweist.

16. Beatmungsgerät (10) zur Hochfrequenzbeatmung insbesondere von neonatalen und pädiatrischen Patienten umfassend ein HF-Modul (1) nach einem der vorhergehenden Ansprüche.

## Claims

1. An HF module (1) for a ventilation device for the high-frequency ventilation particularly of neonatal and pediatric patients (13), wherein the HF module (1) comprises at least one drive unit (3) for generating high-frequency oscillations and at least one HF housing (2), wherein the HF housing (2) comprises an access opening, which can be configured as a tube connection (6), and a membrane (9), wherein the HF housing (2) can be coupled to the drive unit (3) in such a way that the membrane (9) can be displaced by means of the drive unit (3), wherein the HF housing (2) has a coupling means (5) with which the HF housing (2) can be detachably linked to the drive unit (3), **characterised in that** the HF module (1) has at least one further HF housing (2') and at least one further drive unit (3'), wherein each HF housing (2, 2') is associated with one drive unit (3, 3') which acts on the membrane (9, 9') of the associated HF housing (2, 2'), wherein the actuation directions of the drive units (3, 3') are collinear and the drive units (3, 3') are operated in opposite directions, and that the drive units are disposed adjacent to each other in space, so that the drive units can mechanically support each other during the operation of the HF module (1).

2. The HF module (1) according to claim 1, **characterised in that** the coupling means (5) is configured to detachably link the HF housing (2) to the drive unit (3).

3. The HF module (1) according to any one of the preceding claims, **characterised in that** the coupling means (5) has a bayonet coupling, a clamping coupling, a screw coupling, a latching coupling, a flange or a plug-in coupling.

4. The HF module (1) according to claim 3, **characterised in that** the coupling means (5) is configured to be detachable without tools by a user.

5. The HF module (1) according to claim 3 or 4, **characterised in that** a locking mechanism for the coupling means (5) is provided, which has an unlocked and a locked state, wherein the coupling means (5) can be detached exclusively in the unlocked state of the locking mechanism.

6. The HF module (1) according to claim 5, **characterised in that** the locking mechanism is configured as a lock.

7. The HF module (1) according to any one of the claims 5 and 6, **characterised in that** the locking mechanism is configured as a cover which denies the access to the coupling means (5) by a user by covering it at least partially.

8. The HF module (1) according to any one of the preceding claims, **characterised in that**, in the actuated state of the coupling means (5), the HF housing (2) forms an inner space sealed in a germ-proof manner.

9. The HF module (1) according to claim 1, **characterised in that** the HF housing (2) forms an inner space sealed in a germ-proof manner, which is accessible exclusively via the access opening even in the detached state of the coupling means (5).

10. The HF module (1) according to claim 9, **characterised in that** the membrane (9) is formed integrally with the HF housing (2).

11. The HF module (1) according to claim 9, **characterised in that** the membrane (9) is connected to the HF housing (2) in a loss-proof manner.

12. The HF module (1) according to any one of the preceding claims, **characterised in that** the drive units (3, 3') each have one stator (3.1, 3.1') and one actuator (3.2, 3.2'), wherein the stators (3.1, 3.1') of at least two drive units (3), respectively, support each other mechanically.

13. The HF module (1) according to any one of the preceding claims, **characterised in that** the HF housing (2) comprises a material selected from a group comprising at least polysulphone, polyimide, polyether imide, polyethylene, polypropylene, polyurethane and/or polyethylene terephthalate.

14. The HF module (1) according to any one of the preceding claims, **characterised in that** the membrane (9) of the HF housing (2) comprises a material selected from a group comprising at least polyester, polytetrafluoroethylene, silicone and/or polyvinyl chloride.

15. The HF module (1) according to any one of the preceding claims, **characterised in that** the HF housing (2) has an injection-moulded part.

16. A ventilation device (10) for the high-frequency ventilation particularly of neonatal and pediatric patients, comprising an HF module (1) according to any one of the preceding claims.

## Revendications

1. Module HF (1) pour un respirateur pour la respiration artificielle à haute fréquence en particulier de patients (13) néonataux et pédiatriques, dans lequel le module HF (1) comprend au moins une unité d'entraînement (3) destinée à générer des oscillations à haute fréquence et au moins un boîtier HF (2), ledit boîtier HF (2) comprenant une ouverture d'accès qui peut être réalisée en tant que raccord de tuyau flexible (6) ainsi qu'une membrane (9), ledit boîtier HF (2) pouvant être couplé à ladite unité d'entraînement (3) de telle manière que ladite membrane (9) peut être déviée au moyen de l'unité d'entraînement (3), ledit boîtier HF (2) présentant un moyen de couplage (5) par le biais duquel le boîtier HF (2) peut être relié d'une manière amovible à ladite unité d'entraînement (3), **caractérisé par le fait que** ledit module HF (1) présente au moins un autre boîtier HF (2') et au moins une autre unité d'entraînement (3'), à chaque boîtier HF (2, 2') étant associée une unité d'entraînement (3, 3') qui agit sur ladite membrane (9, 9') du boîtier HF (2, 2') associé, les dispositifs de commande des unités d'entraînement (3, 3') étant colinéaires et les unités d'entraînement (3, 3') étant opérées en sens opposé, et que lesdites unités d'entraînement sont disposées de manière à être contiguës dans l'espace de sorte que, en fonctionnement du module HF (1), les unités d'entraînement peuvent s'appuyer mécaniquement l'une sur l'autre.

2. Module HF (1) selon la revendication 1, **caractérisé par le fait que** ledit moyen de couplage (5) est agencé pour relier de manière amovible le boîtier HF (2) à ladite unité d'entraînement (3).

3. Module HF (1) selon l'une quelconque des revendications précédentes, **caractérisé par le fait que** ledit moyen de couplage (5) présente un accouplement à baïonnette, un accouplement de serrage, un accouplement de vissage, un accouplement à encliquetage, une bride ou un accouplement à emboîtement.

4. Module HF (1) selon la revendication 3, **caractérisé par le fait que** ledit moyen de couplage (5) est réalisé de manière à pouvoir être desserré sans outil par un utilisateur.

5. Module HF (1) selon la revendication 3 ou 4, **caractérisé par le fait qu'**un dispositif de verrouillage est prévu pour ledit moyen de couplage (5), qui présente un état d'ouverture et un état de verrouillage, ledit moyen de couplage (5) pouvant être desserré exclusivement en état d'ouverture du dispositif de verrouillage.

6. Module HF (1) selon la revendication 5, **caractérisé par le fait que** ledit dispositif de verrouillage est réalisé en tant que serrure.

7. Module HF (1) selon l'une quelconque des revendications 5 et 6, **caractérisé par le fait que** ledit dispositif de verrouillage est réalisé en tant que cache qui évite qu'un utilisateur n'ait accès audit moyen de couplage (5) en le recouvrant au moins en partie.

8. Module HF (1) selon l'une quelconque des revendications précédentes, **caractérisé par le fait que** ledit boîtier HF (2) forme, en état actionné du moyen de couplage (5), un volume intérieur fermé hermétiquement aux germes.

9. Module HF (1) selon la revendication 1, **caractérisé par le fait que** ledit boîtier HF (2) forme un volume intérieur fermé hermétiquement aux germes qui, également en état desserré du moyen de couplage (5), est accessible exclusivement via ladite ouverture d'accès.

10. Module HF (1) selon la revendication 9, **caractérisé par le fait que** ladite membrane (9) est réalisée d'un seul tenant avec ledit boîtier HF (2).

11. Module HF (1) selon la revendication 9, **caractérisé par le fait que** ladite membrane (9) est reliée d'une manière imperdable audit boîtier HF (2).

12. Module HF (1) selon l'une quelconque des revendications précédentes, **caractérisé par le fait que** lesdites unités d'entraînement (3, 3') présentent chacune un stator (3.1, 3.1') et un actionneur (3.2, 3.2'), lesdits stators (3.1, 3.1') d'au moins respectivement deux unités d'entraînement (3) s'appuyant mécaniquement l'un sur l'autre.

13. Module HF (1) selon l'une quelconque des revendications précédentes, **caractérisé par le fait que** ledit boîtier HF (2) comprend une matière choisie dans un groupe comprenant au moins le polysulfone, les polyimides, les polyéthe-rimides, les polyéthylènes, le polypropylène, les polyuréthanes et/ou le polyéthylène téréphtalate.

14. Module HF (1) selon l'une quelconque des revendications précédentes, **caractérisé par le fait que** ladite membrane (9) du boîtier HF (2) comprend une matière choisie dans un groupe comprenant au moins le polyester, le polytétrafluoréthylène, la silicone et/ou le polychlorure de vinyle.

15. Module HF (1) selon l'une quelconque des revendications précédentes, **caractérisé par le fait que** ledit boîtier HF (2) est une pièce moulée par injection.

16. Respirateur (10) pour la respiration artificielle à haute fréquence en particulier de patients néonataux et pédiatriques, comprenant un module HF (1) selon l'une quelconque des revendications précédentes.
